# EUROPEAN PATENT APPLICATION

(11) **EP 4 282 407 A1**
(43) Date of publication of application: **29.11.2023**
(21) Application number: 22305782.9
(22) Date of filing: 27.05.2022
(51) Int. Cl.: A61K 31/137, A61K 31/661, A61K 45/06, A61P 35/00, A61P 35/02

(54) **TREATMENT OF CANCER WITH S1P RECEPTOR AGONISTS**

(71) Applicant: Priothera SAS, 68300 Saint-Louis (FR)
(72) Inventor: OEHEN, Stephan, 4123 ALLSCHWIL (CH); DERTSCHNIG, Simone, 4054 BASEL (CH)
(74) Representative: Plasseraud IP

(57) **Abstract**

The present disclosure relates to a S1P receptor agonist, for use in treating cancer in a subject in need thereof, wherein said subject does not undergo hematopoietic stem cell transplant (HSCT) for cancer therapy.

## Description

### TECHNICAL FIELD

The present disclosure relates to S1P receptor agonists, preferably mocravimod, for use in treating patients suffering from cancer in patients who do not undergo hematopoietic stem cell transplantation.

### BACKGROUND ART

Cancer remains one of the most devastating diseases in the world with more than 10 million new cases every year. It is a disease that is characterized by uncontrolled cell growth, almost anywhere in the body. The rate of growth of tumor masses is impressive, as tumor cells replicate much faster than healthy cells; hence the need for treatments that are sufficiently effective to counteract the rate of tumor cell development while minimizing the destruction of healthy tissues. Tumor formation is where uncontrolled cell growth occurs in non-solid tissue such as in hematological cancer or in solid tissue such non-hematological cancer which can occur in an organ, muscle, or bone. The treatments currently used to treat cancer are generally intrusive processes with many side effects: chemotherapy to shrink the tumor, followed by surgery to remove it when possible, and additional chemotherapy and radiotherapy.

While rates of cancer survival are increasing through the development of improved therapies, the cancer mortality rate remains high. Improved cancer therapies are therefore the subject of ongoing research and development by the scientific community.

For most non-hematological cancer, surgery to reduce the size of, or eradicate a cancer, may be a therapeutic option. The surgery may also increase the effectiveness of subsequently administered anticancer therapies, such as immunotherapy, chemotherapy and/or radiotherapy. However, surgical intervention in cancer therapy is not without risk. In addition to reproducing uncontrollably, cancer cells detach from a primary tumor during a surgery to travel elsewhere in the body via the circulatory and lymphatic systems. Cancer spread (i.e. metastases) during surgical intervention therefore presents a significant risk to a cancer patient.

Left untreated, hematological cancer may lead to death within weeks or months. Current treatments for hematological cancer include chemotherapy, including targeted chemotherapies, radiation therapy, and autologous hematopoietic stem cell transplantation or allogeneic hematopoietic stem cell transplantation. The side effects of each of these treatments are well documented.

Over the past 25 years, much research has increased the effectiveness of therapies, but these improvements are not yet sufficient. There is still a scarcity of clinically effective treatments for cancer.

In contrast to FTY720 which displays poor selectivity for S1PR1 versus S1PR3-5, the compound KRP203 (mocravimod) acts specifically on S1PR1 with a potentially milder toxicity profile. Yokoyama et al have shown that short-term administration of KRP203 alone induced apoptosis of donor T cells in the secondary lymphoid organs and ameliorated acute GVHD (Yokoyama E, et al. Bone Marrow Transplant. 2020; 55: 787-795).

WO2014/128611 discloses the use of mocravimod for treating acute GVHD. WO2020/022507 discloses the use of mocravimod for treating hematological malignancies in subject undergoing HSCT.

However, to the knowledge of the inventors, the role of the S1P receptor agonists, preferably mocravimod, has never been shown in the treatment of cancer in a patient who does not undergo hematopoietic stem cell transplantation.

Without limiting the disclosure to any particular mechanism, the disclosure described here meets this need, as the described methods provide a synergistic therapeutic effect, treating cancer patient who do not undergo hematopoietic stem cell transplantation.

### SUMMARY

A first aspect of the disclosure relates to a S1P receptor agonist for use in treating cancer in a subject in need thereof, wherein said subject does not undergo hematopoietic stem cell transplant (HSCT) for cancer therapy.

A second aspect of the disclosure pertains to a method for inducing apoptosis in vivo of a tumor cell of a subject, said method comprising administering a sufficient amount of said S1P receptor agonist.

A third aspect of the disclosure relates to a method for treating cancer in a subject in need thereof, said method comprising administering a therapeutically efficient amount of said S1P receptor agonist, optionally in combination with another cancer therapy.

### DETAILED DESCRIPTION

### Definitions

In the following, terms as used herein are defined in their meaning.

The term "about" or "ca." has herein the meaning that the following value may vary for ± 20%, preferably ± 10%, more preferably ± 5%, even more preferably ± 2%, even more preferably ± 1%.

Unless otherwise defined, "%" has herein the meaning of weight percent (wt%), also referred to as weight by weight percent (w/w%).

The terms "patient", "subject", "individual", and the like, are used interchangeably herein, and refer to a human. In some embodiments, the patient, subject or individual in need of treatment includes those who already have the disease, condition, or disorder, i.e. cancer.

As used herein, unless specified otherwise, the term "treating" or "treatment", denotes reversing, alleviating, inhibiting the progress of, or preventing the disorder or condition to which such term applies, or reversing, alleviating, inhibiting the progress of, or preventing one or more symptoms of the disorder or condition to which such term applies. In specific embodiments, the term "treating" or "treatment" in relation to cancer refers to stopping, or inhibiting the growth of a tumor or reducing the size of a tumor.

The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors and benign cancers. The term "cancer" as used herein includes primary malignant cells or tumors, e.g., those whose cells have not migrated to sites in the subject's body other than the site of the original malignancy or tumor, and secondary malignant cells or tumors,e.g., those arising from metastasis, the migration of malignant cells or tumor cells to secondary sites that are different from the site of the original tumor.

As used herein, the terms "effective amount" or "therapeutically efficient amount" of a compound refer to an amount of the compound that will elicit the biological or medical response of a subject, for example, ameliorate the symptoms, alleviate conditions, slow or delay disease progression, or prevent a disease, either as the sole active ingredient, or in combination with other active ingredients.

The term "pharmaceutically acceptable salts thereof" includes both acid and base addition salts. Non-limiting examples of pharmaceutically acceptable acid addition salts include chlorides, hydrochlorides, bromides, sulfates, nitrates, phosphates, sulfonates, methane sulfonates, formates, tartrates, maleates, citrates, benzoates, salicylates, and ascorbates. Non-limiting examples of pharmaceutically acceptable base addition salts include sodium, potassium, lithium, ammonium (substituted and unsubstituted), calcium, magnesium, iron, zinc, copper, manganese, and aluminum salts. Pharmaceutically acceptable salts may, for example, be obtained using standard procedures well known in the field of pharmaceuticals. For mocravimod, pharmaceutically acceptable salts would typically be acid-addition salts, since mocravimod is itself a base. Preferably, the pharmaceutically acceptable salts thereof is hydrochloride salt.

The term "pharmaceutically acceptable excipients" as used herein refers to substances that do not cause substantial adverse allergic or immunological reactions when administered to a subject.

As used herein, HSCT refers to the transplantation of multipotent hematopoietic stem cells, usually derived from bone marrow, peripheral blood, or umbilical cord blood, to replicate inside of a patient to produce additional normal blood cells. In particular, HSCT is performed for subjects with certain hematological cancers, such as multiple myeloma or leukemia. In these cases, the recipient's immune system is usually destroyed with radiation or chemotherapy before the transplantation. Infection and graft-versus-host-disease are major complications of allogeneic HSCT.

### S1P receptor agonist

S1P receptors are divided into five subtypes related G-coupled protein receptors (i.e., S1P1, S1P2, S1P3, S1P4 and S1P5), which are expressed in a wide variety of tissues and exhibit different cell specificity.

The S1P receptor agonist for use according to the present methods of the disclosure is a compound which modulates one or more of the five S1P receptor types 1 to 5 (S1PR1-5) by activating or inhibiting the receptor for signal transduction. Such compounds are also referred to herein as "S1P agonists".

In specific embodiments, said S1P receptor agonist for use in the treatment methods of the present disclosure is selected among KRP203 (mocravimod), FTY720 (fingolimod), siponimod (Mayzent^{®}), fingolimod (Gilenya^{™}), ozanimod (Zeposia^{®}), ponesimod, etrasimod, AKP-11, cenerimod, amiselimod, CBP-307, OPL-307, OPL-002, BMS-986166, SCD-044, BOS-173717, CP-1050. Preferably, said S1P receptor agonist for use in the treatment methods of the present disclosure is KRP203 (mocravimod).

Mocravimod is a S1P receptor agonist, particularly a S1PR1 selective agonist of formula 2-amino-2- [4- (3-benzyloxyphenylthio) -2-chlorophenyl] ethyl-propane-1,3-diol, of formula (II) (also referred as mocravimod or KRP203): 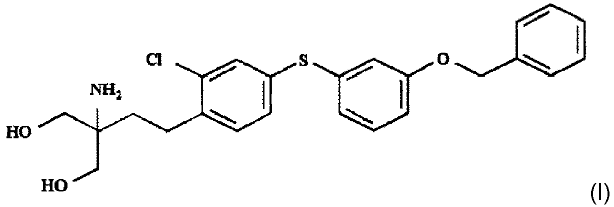 or pharmaceutically acceptable salts thereof.

Phosphate derivatives of mocravimod for use according to the present disclosure, are of formula (Ila) or (IIb): 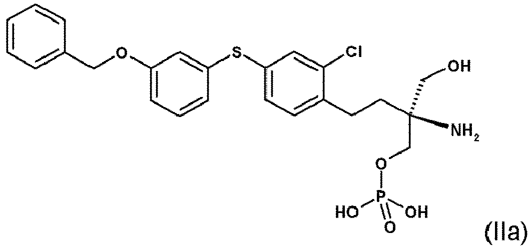 or

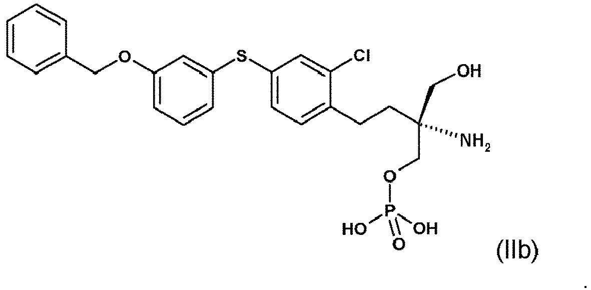

Said compounds and their synthesis methods are also disclosed in WO03/029205, WO2004/074297, WO2006/009092, WO2006/041019 and WO2014128611A1 (which disclosures are incorporated herein by reference).

Mocravimod or KRP203 is particularly preferred.

### Pharmaceutical composition comprising the S1P receptor agonist

The present disclosure also relates to a pharmaceutical composition of said S1P receptor agonist, preferably mocravimod, as described above, in particular for their use in the treatment methods as disclosed in the next section.

In an embodiment, the pharmaceutical composition of the present disclosure comprises the S1P receptor agonist, preferably mocravimod, and one or more pharmaceutically acceptable excipients. Any suitable excipients known to those of ordinary skill in the art for use in pharmaceutical compositions may be employed in the compositions described herein.

The pharmaceutical composition may be administered in any manner appropriate to the disease or disorder to be treated as determined by persons of ordinary skill in the medical arts. An appropriate dose and a suitable duration and frequency of administration will be determined by such factors as discussed herein, including the condition of the patient, the type and severity of the patient's disease, the particular form of the active ingredient, and the method of administration. In general, an appropriate dose (or effective dose) and treatment regimen provides the pharmaceutical composition in an amount sufficient to provide a therapeutic effect, for example, an improved clinical outcome, such as more frequent complete or partial remissions, or longer disease-free and/or overall survival, or a lessening of symptom severity or other benefit as described in detail herein.

The pharmaceutical composition described herein may be administered to a subject in need thereof by any of several routes that can effectively deliver an effective amount of the compound. In an embodiment the pharmaceutical composition is administered by parenteral or enteral route.

When the pharmaceutical composition is administered by parenteral route, it may be administered by injection, that is, using a needle (usually a hypodermic needle) and a syringe, or by the insertion of an indwelling catheter. Examples of parenteral administration include intravenous, intramuscular, intranasal, transdermal, submucosal, intrathecal, subcutaneous, intracapsular, intravaginally, intraperitoneally etc.

When the pharmaceutical composition is administered by enteral route, it may be administered orally, rectally, or bucally. In a preferred embodiment, the pharmaceutical composition is suitable to be administered orally.

In another embodiment, the pharmaceutical composition may be a solid dosage form suitable for oral administration. Solid dosage forms for oral administration include capsules, tablets, pills, powders, and granules. In a preferred embodiment, the pharmaceutical composition is a capsule or a tablet. The capsule may be a soft or hard gelatin capsule, preferably a hard gelatin capsule. For example, the capsule is HGC Crushed or HPMC capsules Crushed.

In an embodiment, the release of the capsule or tablet content may be immediate or modified such as delayed, targeted or extended. In a preferred embodiment the solid dosage form is an immediate release dosage form.

In an embodiment, the pharmaceutical composition comprises S1P receptor agonist, preferably mocravimod, and one or more pharmaceutically acceptable excipients, adjuvants or other pharmaceutically acceptable carriers.

Example of pharmaceutically acceptable excipients are fillers and mixtures thereof, disintegrants and mixtures thereof, lubricants and mixtures thereof and, glidants and mixtures thereof.

In an embodiment, the pharmaceutical composition comprises the S1P receptor modulator, preferably mocravimod, and one or more pharmaceutically acceptable excipients, and particularly, at least one filler and mixtures thereof, a disintegrant, a lubricant and, a glidant.

### Fillers

Fillers, (also referred to as a diluents, dilutants or thinners) are substance which are added to the drug substance in order to make the latter suitable for oral administration (e.g., capsules, tablets). Fillers themselves should not produce any pharmacological effect on human being. Examples of fillers include mannitol, microcrystalline cellulose, lactose monohydrate, anhydrous lactose, corn starch, xylitol, sorbitol, sucrose, dicalcium phosphate, maltodextrin, and gelatin. The pharmaceutical composition of the present disclosure comprises at least one filler selected from mannitol, microcrystalline cellulose and mixtures thereof. In a preferred embodiment, the pharmaceutical composition of the present disclosure comprises a mixture of mannitol and microcrystalline cellulose.

### Disintegrants

Disintegrants are added to oral solid dosage forms to aid in their deaggregation. Disintegrants are formulated to cause a rapid break-up of solids dosage forms when they come into contact with moisture. Disintegration is typically viewed as the first step in the dissolution process. Examples of disintegrants include the modified starch such as sodium starch glycolate, sodium carboxymethyl starch, and pre-gelatinized starch, crosslinked polymers, such as crosslinked polyvinylpyrrolidone (crospovidone) or crosslinked sodium carboxymethyl cellulose (croscarmellose sodium), and calcium silicate. The pharmaceutical composition of the present disclosure comprises sodium starch glycolate as disintegrant.

### Lubricants

Lubricants are substances that we use in tablet and capsule formulations in order to reduce the friction. Lubricant can facilitate extrusion of tablets from matrix, thus preventing formation of scratches on their surfaces. By nature lubricants can be divided into two groups: a) fats and fat-like substances; b) powdery substance. Powdery substances are more applicable then the fat-like ones, because the latter impact on solubility and chemical stability of the tablets. Powdery lubricants are introduced by powdering of granulate. They provide constant-rate outflow of mass for tabletizing from hopper into matrix that guaranties accuracy and constancy of the drug substance dosage.

Examples of lubricants include magnesium stearate, hydrogenated castor oil, glyceryl behenate, calcium stearate, zinc stearate, mineral oil, silicone fluid, sodium lauryl sulfate, L-leucine, and sodium stearyl fumarate. The pharmaceutical composition of the present disclosure comprises magnesium stearate as lubricant.

### Glidants

Glidants are blended with the formulation to enhance the tablet-core blend-material flow property. During the early stage of compression, glidants are mixed within the particle arrangement of the tablet powder blend to improve flowability and uniformity within the die cavity of tablet presses. Glidants encourage the flow of tablet granulation by diminishing friction between particles. The effect of glidants on the flow of the granules depends on the size and shape of the particles of the granules and the glidants. Above a certain concentration, the glidant will in fact function to inhibit flowability. In tablet manufacture, glidants are usually added just prior to compression. Examples of glidants include colloidal silicon dioxide, starch, magnesium stearate and talc. The pharmaceutical composition of the present disclosure comprises colloidal silicon dioxide as lubricant.

The pharmaceutical composition of the present disclosure comprises the S1P receptor agonist, preferably mocravimod, and at least one filler selected from mannitol, microcrystalline cellulose and mixtures thereof, sodium starch glycolate as disintegrant, magnesium stearate as lubricant and, colloidal silicon dioxide as glidant.

Any suitable excipients known to those of ordinary skill in the art in pharmaceutical compositions may be further employed in the compositions described herein.

In an embodiment the dosage strength of the S1P receptor agonist, preferably mocravimod, in the solid dosage form is between 0.05 mg to 15 mg/unit, preferably between 0.1mg to 10mg/unit, for example about 0.1 mg/unit, or about 0.4mg/unit, or about 1 mg/unit, or about 10 mg/unit, more preferably about 1 mg/unit.

More specifically, the pharmaceutical composition of the present disclosure further comprises the following ingredients:
- mannitol, preferably at a content from 48 to 88 mg/unit, more preferably from 58 to 78mg/unit, even more preferably at a content about 68 mg/unit;
- microcrystalline cellulose, preferably at a content from 5 to 45 mg/unit, more preferably from 15 to 35 mg/unit, even more preferably at a content about 25 mg/unit;
- sodium starch glycolate, preferably at a content from 1 to 8 mg/unit, more preferably from 2 to 6 mg/unit, even more preferably at a content about 4 mg/unit;
- magnesium stearate, preferably at a content from 0.025 to 4 mg/unit, more preferably from 0.5 to 2 mg/unit, even more preferably at a content about 1 mg/unit; and
- colloidal silicon dioxide, preferably at a content from 0.125 to 2 mg/unit, more preferably from 0.25 to 1 mg/unit, even more preferably at a content about 0.5 mg/unit.

In another embodiment, the pharmaceutical composition of the present disclosure comprises the following ingredients:
- S1P receptor agonist, preferably mocravimod, preferably at a content from 0.05% to 15%, more preferably from 0.1% to 10% mg/unit, even more preferably at a content about 0.1% or 0.4% or 1% or 10%;
- mannitol, preferably at a content from 48% to 88%, more preferably from 58% to 78%, even more preferably at a content about 68%;
- microcrystalline cellulose, preferably at a content from 5% to 45%, more preferably from 15% to 35%, even more preferably at a content about 25%;
- sodium starch glycolate, preferably at a content from 1% to 8%, more preferably from 2% to 6%, even more preferably at a content about 4%;
- magnesium stearate, preferably at a content from 0.025% to 4%, more preferably from 0.5% to 2%, even more preferably at a content about 1%;
- colloidal silicon dioxide, preferably at a content from 0.125 to 2 mg/unit, more preferably from 0.25 to 1 mg/unit, even more preferably at a content about 0.5 mg/unit,

Percentage being expressed mg/mg of the total composition in dry weight.

In an embodiment, the pharmaceutical composition of the disclosure is stable for at least 1 month at 50°C, preferably 2 months at 50°C.

In another embodiment, the pharmaceutical composition of the disclosure is stable at least 24 months at 5°C.

In another embodiment, the pharmaceutical composition of the disclosure is stable at least 24 months at 25°C/ 60% relative humidity.

As used herein, stability of a composition is measured according to the following method: high-pressure liquid chromatography (HPLC) which is widely known in the field. A composition is stable if the sum of impurities is less than or equal to 0.7% with a confidence interval of [98-102]%.

In a specific embodiment the pharmaceutical composition is prepared according to the following steps:
a. Blending the S1P receptor modulator with microcrystalline cellulose, colloidal silicon dioxide, preferably at 22rpm for 18 mins,
b. adding mannitol and blending the resulting mixture, preferably at 22rpm for 9 mins,
c. adding sodium starch glycolate and blending the resulting mixture, preferably at 22rpm for 5 mins,
d. adding magnesium stearate, blending the resulting pharmaceutical composition, preferably at 22rpm for 5 mins,
e. recovering the pharmaceutical composition of the present disclosure.

In a preferred embodiment, the process further comprises the step of:
f. filling the resulting pharmaceutical composition into capsules,
g. recovering the resulting capsules filled with the pharmaceutical composition.

### The patient population to be preferably targeted by the treatment methods

The inventors have surprisingly shown that the S1P receptor agonist, preferably mocravimod, induces apoptosis in cancer cells, and may therefore be used as anticancer drugs, when administering a sufficient amount of S1P receptor agonist, preferably mocravimod to induce apoptosis of tumor cells in a subject in need thereof, irrespective whether the subject is undergoing hematopoietic stem cell transplant for cancer treatment.

Accordingly, in specific embodiments, the subject that is targeted by the treatment methods disclosed herein is a subject who has cancer and who does not undergo HSCT.

In other specific embodiments, which can be combined with the previous embodiment, the subject that is targeted by the treatment methods disclosed herein is a subject who has cancer and who does not receive CAR-cell therapy. For example, in particular embodiments, said subject has a cancer which cannot be treated by HSCT, or said subject is not eligible for HSCT therapy.

Said cancer encompass solid and liquid, e.g., diffuse or circulating, cancer and includes malignancies of the various organ systems such as hematological or non-hematological cancer.

Cancers that may be treated include tumors that are not vascularized, or not yet substantially vascularized, as well as vascularized tumors. The cancers may comprise non-solid tumors (such as hematological tumors, for example, leukemias and lymphomas including relapses and treatment-related tumors e.g. secondary malignancies after hematopoietic stem cell transplantation (HSCT)) or may comprise solid tumors.

Examples of cancers include, but are not limited to, hematological malignancies such as large B-Cell Lymphoma (DLBCL), chronic myeloid leukemia (CML), acute myeloid leukemia (AML), chronic lymphocytic leukemia (CLL), acute lymphocytic leukemia (ALL), Hodgkin lymphoma, non-Hodgkin lymphoma, Mantle cell lymphoma (MCL), primary mediastinal large B-cell lymphoma (PMBCL) or multiple myeloma.

In specific embodiments, said cancer is a non-hematological cancer, with solid tumor, for example selected from the group consisting of colon cancer, breast cancer, lung cancer, brain cancer, prostate cancer, head and neck cancer, pancreatic cancer, bladder cancer, colorectal cancer, bone cancer, cervical cancer, ovarian cancer, liver cancer, oral cancer, esophageal cancer, thyroid cancer, kidney cancer, stomach cancer, testicular cancer and skin cancer.

### The methods of use

The present disclosure also relates to the use of a S1P receptor agonist, preferably mocravimod, as disclosed in the previous section, and their pharmaceutical compositions, in methods for inducing *in vivo* apoptosis of a tumor cell in a subject, said method comprising administering a sufficient amount of S1P receptor agonist, preferably mocravimod for inducing apoptosis in vivo of said tumor cell.

The S1P receptor agonist, preferably mocravimod, as disclosed in the previous section, and their pharmaceutical compositions, are also useful as a drug in methods for treating cancer in a subject, said method comprising administering a therapeutically efficient amount of the S1P receptor agonist, preferably mocravimod, and more particularly in the sub-population of patients as defined in the previous section, in particular in subjects not eligible for HSCT therapy and/or CAR-cells therapy, preferably CAR-T cells therapy.

To perform the methods of treatment as disclosed herein, an effective amount of the S1P receptor agonist, preferably mocravimod, is administered to the subject in need of such treatment.

"An effective amount" or "therapeutically efficient amount" as used herein, refers to the amount of the S1P receptor agonist, preferably mocravimod, required to confer therapeutic effect on the subject, either alone or in combination with one or more other active agents. Effective amounts vary, as recognized by those skilled in the art, depending on, for example, route of administration, excipient usage, and co-usage with other active agents. In the case of treating a particular disease or condition, the desired therapeutic effect is inhibiting the progression of the disease. This may involve only slowing the progression of the disease temporarily, although more preferably, it involves halting the progression of the disease permanently. This can be monitored by routine methods or can be monitored according to diagnostic methods discussed herein. The desired response to treatment of the disease or condition also can be delaying the onset or even preventing the onset of the disease or condition.

In specific embodiments, the desired response can be inducing in vivo apoptosis of tumor cells.

In other specific embodiments, the desired response can be treating cancer, or delaying or preventing the onset of cancer, relapse cancer or death.

In other embodiments, the desired response can be increasing survival of the subject as compared to without administration of the S1P receptor agonist, preferably mocravimod.

In other embodiments, the desired response can be improving the quality of life of the subject as compared to without administration of the S1P receptor agonist, preferably mocravimod.

The S1P receptor agonist, preferably mocravimod, may be daily administered for short period, for example 1 day, 7 days, 14 days, 21 days, 28 days or 1 month, or for a longer period (as chronic use), for example for at least 1, 2, 3, 4, 5, 6, 7, 8, 9, 10, 11, 12, 18, 24 months, or more, typically during the life of the subject, or until relapse.

The daily dosage amount and dose of the S1P receptor agonist, preferably mocravimod, described herein may depend upon the subject's condition, that is, stage of the disease, severity of symptoms caused by the disease, general health status, as well as age, gender, and weight, and other factors apparent to a person of ordinary skill in the medical art. Similarly, the dose of S1P receptor agonist, preferably mocravimod, for inducing apoptosis of tumor cells in a subject or for treating cancer in a subject suffering from cancer may be determined according to parameters understood by a person of ordinary skill in the medical art.

In some embodiments, the amount of the S1P receptor agonist, preferably mocravimod, administered per day is a fixed amount. In some embodiments, the fixed daily dosage is 0,05 mg to 40 mg per day, preferably 0,1 mg to 35 mg, more preferably 0,5 mg to 30 mg, even more preferably 1 mg to 15 mg per day, even more preferably 1,5 mg to 7mg, even more preferably 2 mg to 5 mg, even more preferably about 3 mg per day.

In some embodiments, the S1P receptor agonist, preferably mocravimod, is daily administered for at least 1, 2, 3, 4, 5, 6, 7, 8, 9, 10, 11, 12, 18, 24 months, or more, at a dosage of 3 mg per day.

The daily dosage may be administered as one dose per day or in multiple doses in a single day. In a preferred embodiment, the daily dosage is administered once a day. In some embodiments, the doses are administered several times daily, preferably 3 times daily. The minimum dose that is enough to provide effective therapy may be used in some embodiments.

In another embodiment, said S1P receptor agonist, preferably mocravimod is administered at a daily dose of 3mg, e.g. three solid dosage form a day of 1mg. Indeed, 3 solid dosage form, such as capsules or tablets, of 1mg administered in a spaced-apart manner is easier to swallow than a single solid dosage form of 3 mg.

The S1P receptor agonist, preferably mocravimod, may also be used alone and/or in appropriate association, as well as in combination, with other pharmaceutically active compounds.

By "combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapies can be administered in any order. In general, each therapeutic agent will be administered at a dose and/or using a regimen determined for that therapeutic agent. It will further be appreciated that the therapeutic agents utilized in this combination may be administered together in a single composition or administered separately in different compositions. In some embodiments, the therapeutic agents utilized in combination can be utilized at levels that do not exceed the levels at which they are typically utilized individually. In some embodiments, the levels of the therapeutic agents utilized in combination can be lower than those utilized individually. In specific embodiment the S1P receptor agonist, preferably mocravimod, is used in combination with one or more anti-cancer agent(s) and/or radiotherapy. Examples of anti-cancer agents may be antineoplastic agents such as chemotherapy or immuno-oncology therapeutic agents.

Suitable antineoplastic agents may include without limitation:
- alkylating agents such as cyclophosphamide, mechloretamine, chlorambucil, melphalan, nitrosureas, temozolomide,
- anthracyclines such as daunorubicin, doxorubicin, epirubicin, idarubicin, mitoxantrone, valrubicin,
- taxanes such as Paclitaxel, Docetaxel,
- epothilones,
- inhibitors of Topoisomerase I such as Irinotecan or Topotecan,
- inhibitors of Topoisomerase II such as Etoposide, teniposide, or Tafluposide,
- nucleotide analogs and precursor analogs such as azacitidine, azathioprine, capecitabine, cytarabine, flurouracil, gemcitabine, hydroxyurea, mercaptopurine, methotrexate, or Tioguanine,
- peptide antibiotics such as carboplatin, cisplatin and oxaliplatin,
- retinoids such as tretinoin, alitretinoin, bexarotene,
- vinca alkaloids and derivatives such as vinblastine, vincristine, vindesine, vinorelbine,
- targeted therapies such as kinase inhibitors such as Ibrutinib, Idelalisib, Erlotinib, Gefitinib, Imatinib, Vemurafenib, Vismodegib,
- proteasome inhibitors such as bortezomib, carfilzomib, and
- histone deacetylase inhibitors such as Vorinostat or Romidepsin.

Examples of chemotherapy are immunosuppressive agents which include without limitation ciclosporin A, sirolimus, tacrolimus, methotrexate, and mycophenolate. In an embodiment, the immunosuppressant agent comprises or essentially consists of an efficient amount of ciclosporin A. For example, intravenous administration of ciclosporin A may be carried out at an initial dosage of between 2 to 6 mg/kg/day, preferably 3 to 5 mg/kg/day. Dosage adjustments are carried out, based on the toxicity or the concentration of ciclosporin A relative to the target trough concentration (150 to 400 mg / L). The administration of ciclosporin A can be changed to oral administration if the patient can tolerate the oral administration. The initial dosage for oral administration may be set to the current dosage for intravenous administration. The dosage of ciclosporin A is monitored at least weekly and changed to a clinically appropriate dosage. Preferably, when the change to oral maintenance therapy is made, the daily doses of cyclosporin A may be about 12.5 mg/kg.

In the case where methotrexate is used together therewith, the dosing schedule and the dosage of methotrexate will be adapted to the hospital standards. For example, from 6 to 10 mg/kg of methotrexate is administered. Calcineurin inhibitors such as ciclosporin A (CsA) and tacrolimus (TAC) suppress donor T-cell activation and remain the most commonly used immunosuppressants.

Examples of immuno-oncology therapeutic agents are CTLA-4 inhibitors, PD-1 inhibitors, PD-L1 inhibitors, CD52 inhibitor, CD20 inhibitor, GD2 inhibitor, CD19 inhibtors, CD19/CD3 inhibitor, CD38 inhibitor, SLAMF7 inhibitors, EGFR inhibitors, EpCAM inhibitors, HER2 inhibitors, PDGF-Ra inhibitor, VEGF inhibitors, VEGFR2 inhibitors, TLR7 inhibitors, IL-R6 inhibitors, CD22 inhibitors, CD30 inhibitors, CD33 inhibitors, CD123 inhibitors, CD79b inhibitors, nectin-4 inhibitors, Trop-2 inhibitors, IL2R inhibitors, IFNAR1 and IFNAR2 inhibitors .

In another embodiment, the S1P receptor agonist, preferably mocravimod, for use according to the present disclosure has
- maximum plasma levels (Cmax) of between 0.4 ng/mL and 1 ng/mL, preferably between 0.55 ng/mL and 0.85 ng/mL, more preferably between 0.65 ng/mL and 0.75 ng/mL,
- time to maximum plasma level (Tmax) between 2h and 10h, preferably between 4h and 8h, more preferably between 5h and 7h; and,
- area under the plasma concentration curve (AUClast) of between 60h*ng/mL and 74h*ng/mL, preferably between 64h*ng/mL and 70 h*ng/mL, more preferably between 66.5 h*ng/mL and 67.5 h*ng/mL.

In another embodiment, the S1P receptor agonist, preferably mocravimod, for use according to the present disclosure has
- maximum plasma levels (Cmax) between 0.6 ng/mL and 1.20 ng/mL, preferably between 0.75 ng/mL and 1.05 ng/mL, more preferably between 0.85 ng/mL and 0.95 ng/mL,
- time to maximum plasma level (Tmax) between 6h and 14h, preferably between 8h and 12h, more preferably between 9h and 11h, and
- area under the plasma concentration curve (AUClast) of between 63.5 h*ng/mL and 77.5 h*ng/mL, preferably between 67.5 h*ng/mL and 73.5 h*ng/mL, more preferably between 69.5 h*ng/mL and 71.5 h*ng/mL

In another embodiment, the S1P receptor agonist, preferably mocravimod, for use according to the present disclosure has a half time (T ½) of between 110h and 134h, preferably between 116h and 128h, more preferably between 121h and 123h.

In another embodiment, the S1P receptor agonist, preferably mocravimod, for use according to the present disclosure has a half time (T ½) of between 100h and 122h, preferably between 105h and 117h, more preferably between 110h and 112h.

### FIGURES

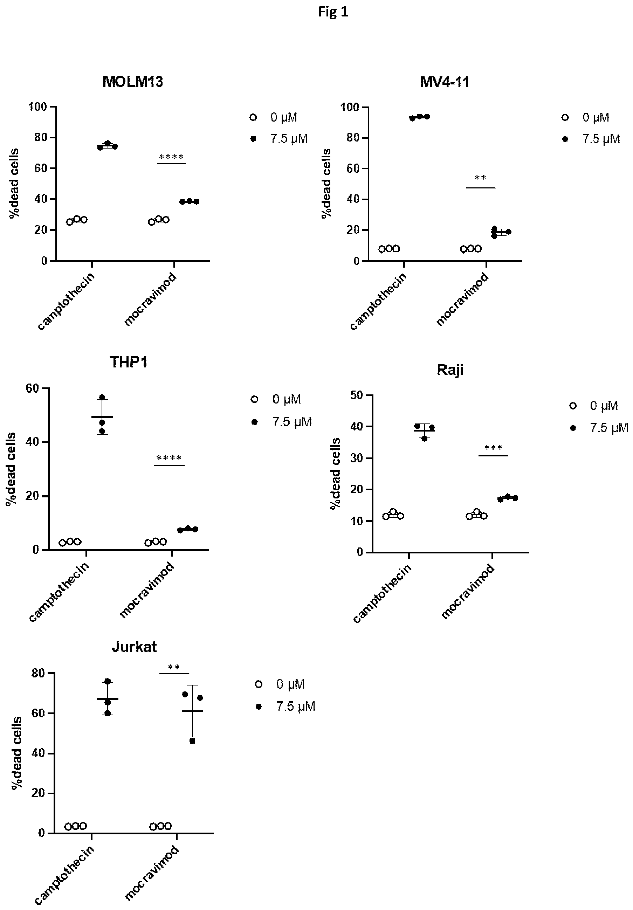

**Figure 1****:** Mocravimod kills tumor cells in vitro. An unpaired t test was used to compare no mocravimod to 7.5µM mocravimod. **p<0.005, ***p<0.001, ****p<0.0001.

### EXAMPLES

Hereinafter, the present disclosure is described in more details and specifically with reference to the examples, which however are not intended to limit the present disclosure.

### Example 1: S1P receptor agonist induces apoptosis

### Material & methods

To investigate cellular cytotoxicity (induction of cell death) of mocravimod on different blood cancer cell lines, cell lines were expanded and incubated for 2 and 24 hours in the presence of 7.5 µM mocravimod. The following cell lines were used: MOLM-13, THP-1, Kasumi-1, Jurkat, Raji, JEKO-1 and MV-4-11. 10⁵ tumor cells were seeded per well in a 96 well plate. 2 and 24hrs post incubation at 37°C, 5% CO2 cells were stained with Annexin V and DAPI to evaluate cell death.

### Results

The percentage of dead cells of each tumor cell line MOLM-13 (AML), THP-1 (AML), Kasumi-1 (AML), Jurkat (ALL), Raji (B cell lymphoma), JEKO-1 (Mantle cell lymphoma) and MV-4-11 (AML) is higher when mocravimod is administered in the treated arm compared to the control untreated arm (Figure 1). Thus, mocravimod induces apoptosis of tumor cells in all MOLM-13, THP-1, Kasumi-1, Jurkat, Raji, JEKO-1 and MV-4-11 cell lines. Therefore, the administration of mocravimod allows to treat cancer by inducing in vivo apoptosis of tumor cells.

## Claims

1. A S1P receptor agonist, for use in treating cancer in a subject in need thereof, wherein said subject does not undergo hematopoietic stem cell transplant (HSCT) for cancer therapy.

2. The S1P receptor agonist for use of Claim 1, which is Mocravimod of formula (I) or pharmaceutically acceptable salts thereof, or phosphate derivates of formula (Ila) or of formula (IIb): 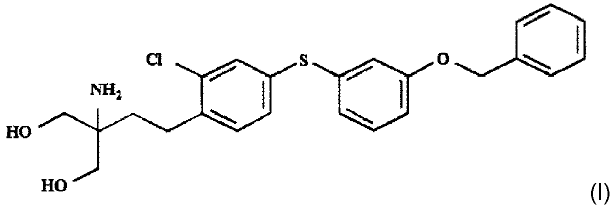 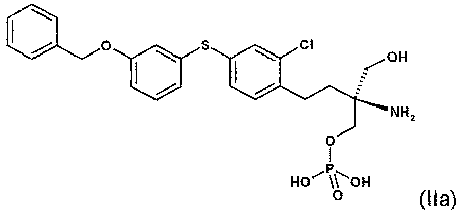 or

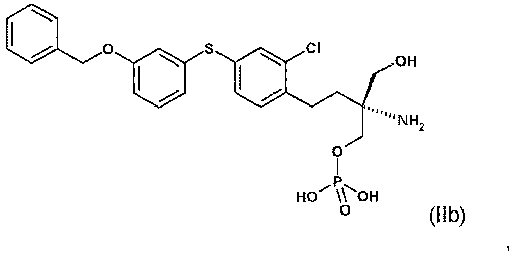

3. The S1P receptor agonist, for use according to claim 1 or 2, wherein the subject further does not receive CAR-cells therapy, preferably CAR-T cells therapy, in combination with said S1P receptor agonist.

4. The S1P receptor agonist, for use according to claims 1, 2 or 3, wherein said cancer is a hematological or a non-hematological cancer.

5. The S1P receptor agonist, for use according to claim 4, wherein said cancer is a hematological cancer selected from the group consisting of large B-Cell Lymphoma (DLBCL), chronic myeloid leukemia (CML), acute myeloid leukemia (AML), chronic lymphocytic leukemia (CLL), acute lymphocytic leukemia (ALL), Hodgkin lymphoma, non-Hodgkin lymphoma, Mantle cell lymphoma (MCL), primary mediastinal large B-cell lymphoma (PMBCL) or multiple myeloma.

6. The S1P receptor agonist for use according to claim 4, wherein said cancer is a non-hematological cancer selected from the group consisting of colon cancer, breast cancer, lung cancer, brain cancer, prostate cancer, head and neck cancer, pancreatic cancer, bladder cancer, colorectal cancer, bone cancer, cervical cancer, ovarian cancer, liver cancer, oral cancer, esophageal cancer, thyroid cancer, kidney cancer, stomach cancer, testicular cancer and skin cancer.

7. The S1P receptor agonist, for use according to any one of claims 1 to 6, wherein said S1P receptor agonist is mocravimod and is administered in an amount sufficient for treating said cancer.

8. The S1P receptor agonist, for use according to any one of claims 1 to 6, wherein said S1P receptor agonist is mocravimod and is administered in an amount sufficient for inducing tumor cell apoptosis.

9. The S1P receptor agonist, for use according to any one of claims 1 to 8, wherein said S1P receptor agonist is mocravimod and is administered by parenteral or enteral route.

10. The S1P receptor agonist, for use according to claim 9, wherein when said S1P receptor agonist is administered by enteral route, it is administered orally at a daily dose of 3mg e.g. 1 dose of 3 mg or three dose of 1mg.

11. The S1P receptor agonist, for use according to any one of claims 1 to 10, wherein when said S1P receptor agonist is administered orally, it is administered as a solid dosage form of 3mg or three solid dosage form of 1mg.

12. A method for inducing apoptosis in vivo of a tumor cell of a subject, said method comprising administering a sufficient amount of said S1P receptor agonist.

13. A method for treating cancer in a subject in need thereof, said method comprising administering a therapeutically efficient amount of said S1P receptor agonist, optionally in combination with another cancer therapy.

14. The method of claim 13, wherein said subject is not eligible for HSCT therapy.

15. The method of claim 13 or 14, wherein said subject is not eligible for CAR-cells therapy.
